# EUROPEAN PATENT APPLICATION

(11) **EP 2 687 520 A1**
(43) Date of publication of application: **22.01.2014**
(21) Application number: 12760759.6
(22) Date of filing: 28.02.2012
(51) Int. Cl.: C07D 275/04, B01J 31/02, C07C 319/14, C07C 323/62, C07B 61/00

(54) **METHOD FOR PRODUCING 1,2-BENZISOTHIAZOL-3-ONE COMPOUND**

(30) Priority: 18.03.2011 JP 2011061361
(71) Applicant: Sumitomo Seika Chemicals Co., Ltd., Kako-gun, Hyogo 675-0145 (JP)
(72) Inventor: KANDA, Hisaaki, Kako-gun Hyogo 675-0145 (JP); KONISHI, Hirotsugu, Kako-gun Hyogo 675-0145 (JP); YAMAGUCHI, Hiroki, Kako-gun Hyogo 675-0145 (JP)
(74) Representative: Weinberger, Rudolf
(86) International application number: PCT/JP2012/054893
(87) International publication number: WO 2012/127996

(57) **Abstract**

The present invention provides a method for producing a 1,2-benzisothiazol-3-one compound by reacting a 2-(alkylthio)benzonitrile compound with a halogenating agent in the presence of water, wherein an alkyl halide that is generated as by-product is reacted with a sulfide to form an alkylthiol, which is converted into an alkali metal salt, and then the resulting alkali metal salt is reacted with a 2-halobenzonitrile compound to be converted into a 2-(alkylthio)benzonitrile compound and reused as a starting material for the production of a 1,2-benzisothiazol-3-one compound. By means of the present invention, it is possible to efficiently use the by-product that is generated during the production of a 1,2-benzisothiazol-3-one compound and economically produce a 1,2-benzisothiazol-3-one compound without placing a burden on the environment.

## Description

### Technical Field

The present invention relates to a method for producing 1,2-benzisothiazol-3-one compounds useful as antimicrobial agents, antifungal agents, etc.

### Background Art

As an example of a known method for producing a 1,2-benzisothiazol-3-one compound, a 2-(alkylthio)benzonitrile compound is reacted with a halogenating agent in the presence of water (Patent Literature 1).

However, in this method, a harmful alkyl halide is generated during the reaction and needs to be removed. This requires a detoxifying system and increases waste water, etc., thus making it undesirable from an environmental and/or economical viewpoint.

### Citation List

Patent Literature
PTL 1: JP8-134051A

### Summary of Invention

### Technical Problem

The present invention has been accomplished in view of the drawbacks of the prior art techniques described above. A major object thereof is to provide a method for producing a 1,2-benzisothiazol-3-one compound in an economically advantageous manner without placing a burden on the environment by effectively using the by-product generated in the production of a 1,2-benzisothiazol-3-one compound.

### Solution to Problem

The present inventors conducted extensive studies to achieve the above object and found that, by subjecting an alkyl halide, which is generated as a by-product in the process of producing a 1,2-benzisothiazol-3-one compound, to a specific process, it can be converted to a 2-(alkylthio)benzonitrile compound, which is the starting material for producing a 1,2-benzisothiazol-3-one compound. The present inventors further found that by reusing the 2-(alkylthio)benzonitrile compound obtained by this method as a starting material for a 1,2-benzisothiazol-3-one compound, a 1,2-benzisothiazol-3-one compound can be produced in an economically advantageous manner by a simple method with a reduced environmental burden. The present invention has thus been accomplished.

More specifically, the present invention provides a method for producing a 1,2-benzisothiazol-3-one compound as described below.
Item 1. A method for producing a 1,2-benzisothiazol-3-one compound represented by formula (2): 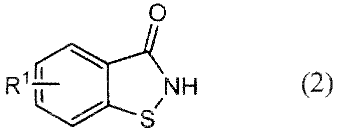 wherein A¹ is a hydrogen atom, a C₁₋₄ alkyl group, a C₁₋₄ alkoxy group, a nitro group, a carboxyl group, an alkoxycarbonyl group, or a halogen atom;
   the method comprising reacting a 2-(alkylthio)benzonitrile compound represented by formula (1): 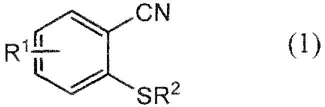 wherein R¹ is as defined above and R² is a C₁₋₄ alkyl group, with a halogenating agent in the presence of water;
   wherein an alkyl halide generated as a by-product in the above reaction and represented by formula (3): R²X¹, wherein R² is as defined above and X¹ is a halogen atom, is subjected to step (i) and step (ii) described below to obtain a 2-(alkylthio)benzonitrile compound represented by formula (1) above, and the resulting 2-(alkylthio)benzonitrile compound is reused as a starting material for producing the 1,2-benzisothiazol-3-one compound:
   (i) reacting the alkyl halide represented by formula (3): R²X¹, wherein R² and X¹ are as defined above, with at least one sulfide selected from the group consisting of alkali metal hydrogensulfide and alkali metal sulfide to form an alkylthiol represented by formula (4): R²SH, wherein R² is as defined above, and then converting the alkylthiol into an alkali metal salt of alkylthiol; and
   (ii) reacting the alkali metal salt of alkylthiol obtained in step (i) with a 2-halobenzonitrile compound represented by formula (5): 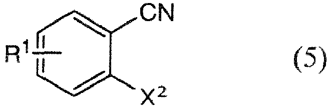
   wherein R¹ is as defined above and X² is a halogen atom, to obtain the 2-(alkylthio)benzonitrile compound represented by formula (1).
Item 2. The method according to Item 1, wherein the reaction of step (i) is conducted in the presence of water and a phase transfer catalyst.
Item 3. The method according to Item 1 or 2, wherein the reaction of step (ii) is conducted in the presence of water and a phase transfer catalyst.
Item 4. The method according to any one of Items 1 to 3, wherein the sulfide used in step (i) is an alkali metal hydrogensulfide.
Item 5. The method according to any one of Items 1 to 4, wherein R¹ is a hydrogen atom and R² is a methyl group in formula (1).
Item 6. A method for producing a 2-(alkylthio)benzonitrile compound represented by formula (1): 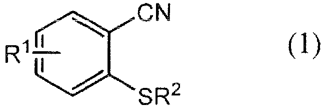 wherein R¹ is a hydrogen atom, a C₁₋₄ alkyl group, a C₁₋₄ alkoxy group, a nitro group, a carboxyl group, an alkoxycarbonyl group, or a halogen atom, and R² is a C₁₋₄ alkyl group;
   the method comprising:
   reacting an alkyl halide represented by formula (3): R²X¹, wherein R² is as defined above and X¹ is a halogen atom, with at least one sulfide selected from the group consisting of alkali metal hydrogensulfide and alkali metal sulfide to obtain an alkylthiol represented by formula (4): R²SH, wherein R² is as defined above,
   converting the alkylthiol into an alkali metal salt of alkylthiol, and then
   reacting the resulting alkali metal salt of alkylthiol with a 2-halobenzonitrile compound represented by formula (5): 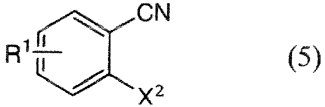
   wherein R¹ is as defined above and X² is a halogen atom.

The present invention is explained in detail below.

The present invention relates to a method for producing a 1,2-benzisothiazol-3-one compound represented by formula (2): 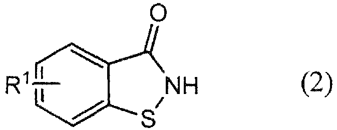 wherein R¹ is a hydrogen atom, a C₁₋₄ alkyl group, a C₁₋₄ alkoxy group, a nitro group, a carboxyl group, an ester thereof, or a halogen atom;
comprising reacting a 2-(alkylthio)benzonitrile compound represented by formula (1): 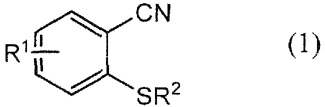 wherein R¹ is as defined above and R² is a C₁₋₄ alkyl group, with a halogenating agent in the presence of water,
wherein an alkyl halide, that is generated as a by-product in the above reaction and represented by formula (3):R²X, wherein R² is as defined above and X is a halogen atom, is reused as a starting material for the reaction described above.

First, the production process of the 1,2-benzisothiazol-3-one compound represented by formula (2) is explained below.

### (1) Production process of 1,2-benzisothiazol-3-one compound

Described below are the groups represented by R¹ in the compounds represented by formula (1): 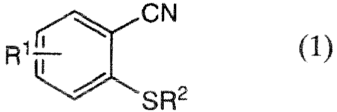 wherein R¹ is a hydrogen atom, a C₁₋₄ alkyl group, a C₁₋₄ alkoxy group, a nitro group, a carboxyl group, an alkoxycarbonyl group, or a halogen atom, and R² is a C₁₋₄ alkyl group. Examples of C₁₋₄ alkyl groups include linear or branched C₁₋₄ alkyl groups, such as a methyl group, an ethyl group, an n-propyl group, an isopropyl group, an n-butyl group, an isobutyl group, a sec-butyl group, and a tert-butyl group. Examples of C₁₋₄ alkoxy groups include a methoxy group, an ethoxy group, an n-propoxy group, an isopropoxy group, an n-butoxy group, an isobutoxy group, a sec-butoxy group, and a tert-butoxy group. Examples of alkoxycarbonyl groups include alkoxycarbonyl groups having a C₁₋₄ linear or branched alkyl group, such as a methoxycarbonyl group, an ethoxycarbonyl group, a propoxycarbonyl group, and a butoxycarbonyl group. Examples of halogen atoms include a chlorine atom and a bromine atom.

Among these groups and atoms represented by R¹, a hydrogen atom, a methyl group, an ethyl group, a tert-butyl group, a methoxy group, a methoxycarbonyl group, an ethoxycarbonyl group, a propoxycarbonyl group, a chlorine atom, a nitro group and the like are preferable, and a hydrogen atom is particularly preferable.

C₁₋₄ alkyl groups represented by R² are the same as alkyl groups exemplified in the groups represented by R¹, among which a methyl group, an ethyl group, an n-propyl group, a tert-butyl group, etc., are preferable, and a methyl group is particularly preferable.

Specific examples of 2-(alkylthio)benzonitrile compounds represented by formula (1) include 2-(methylthio)benzonitrile, 2-(ethylthio)benzonitrile, 2-(n-propylthio)benzonitrile, 2-(tert-butylthio)benzonitrile, 3-methyl-2-(methylthio)benzonitrile, 5-tert-butyl-2-(methylthio)benzonitrile, 4-methoxy-2-(methylthio)benzonitrile, 3-nitro-2-(methylthio)benzonitrile, 3-nitro-2-(tert-butylthio)benzonitrile, 4-chloro-2-(methylthio)benzonitrile, 4-carboxy-2-(methylthio)benzonitrile, and 4-methoxycarbonyl-2-(methylthio)benzonitrile. Among these, 2-(methylthio)benzonitrile, 2-(ethylthio)benzonitrile, 2-(n-propylthio)benzonitrile, and 2-(tert-butylthio)benzonitrile are preferable, and 2-(methylthio)benzonitrile is more preferable because they are readily available and can render high antimicrobial activity to the products.

Examples of usable halogenating agents include chlorine, bromine, sulfuryl chloride, and sulfuryl bromide. Among these, chlorine, sulfuryl chloride and the like are preferable from an economical viewpoint.

The aforementioned 2-(alkylthio)benzonitrile compound represented by formula (1): 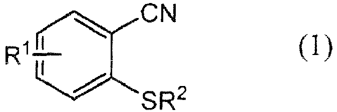 wherein R¹ and R² are as defined above, may be reacted with a halogenating agent in the presence of water, if necessary using a reaction solvent.

In this method, the halogenating agent is preferably used in an amount of about 0.8 to 3 mol, and more preferably about 1 to 2 mol, per mol of 2-(alkylthio)benzonitrile compound. When the amount of the halogenating agent is less than the above range, the amount of unreacted 2-(alkylthio)benzonitrile compound tends to increase, and the yield may be undesirably lowered. When the amount of the halogenating agent is unduly large, side reactions easily occur and the yield may be lowered.

Water is preferably used in an amount of about 0.8 to 5 mol, and more preferably about 1 to 3 mol, per mol of 2-(alkylthio)benzonitrile compound. When the amount of water falls outside this range, side reactions easily occur and the yield may be undesirably lowered.

Water may be used in the form of an aqueous solution of mineral acid by adding a mineral acid to water. Examples of mineral acids include hydrochloric acid, sulfuric acid, and nitric acid. The concentration of the aqueous solution of mineral acid is not particularly limited. In the case of hydrochloric acid, the preferable range generally employed is from 10% by weight to a saturated concentration. In the case of sulfuric acid or nitric acid, 10 to 50% by weight is preferably employed. The addition of mineral acid to water improves selectivity during reaction and suppresses by-product generation.

In the method of the present invention, the use of a reaction solvent is not always necessary; however, a reaction solvent may be used if necessary. The use of a reaction solvent can often make the reaction proceed more smoothly.

The reaction solvent is not particularly limited and any nonaqueous solvent can be used as long as it is inactive to the reaction. Specific examples of such reaction solvents include hydrocarbons such as n-hexane, n-heptane, cyclohexane, methylcyclohexane, benzene, toluene, and xylene; halogenated hydrocarbons such as methylene chloride, 1,2-dichloroethane, chlorobenzene; etc.

When the same reaction solvent as that used in the reaction between the 2-halobenzonitrile compound represented by formula (5) and an alkylthiol described later is used, a 1,2-benzisothiazol-3-one compound can be efficiently obtained in a one-pot process by obtaining a 2-(alkylthio)benzonitrile compound represented by formula (1) via this reaction, and reacting it with the halogenating agent.

When a reaction solvent is used, the amount of the reaction solvent may be generally about 20 to 3,000 parts by mass relative to 100 parts by mass of a 2-(alkylthio)benzonitrile compound. When the amount of the reaction solvent is unduly small, the effect of the addition of the reaction solvent cannot be satisfactorily achieved. When the amount of the reaction solvent is unduly large, the volume efficiency may be undesirably lowered.

The reaction between the 2-(alkylthio)benzonitrile compound represented by formula (1) and a halogenating agent is generally conducted at a temperature of about -20 to 170°C, and preferably about 0 to 150°C. An unduly low reaction temperature is not preferable as this may slow down the reaction speed and prolong the time necessary for the reaction. In contrast, an unduly high reaction temperature may easily cause side reactions and is therefore undesirable.

The reaction time depends on the reaction temperature, reaction solvent, etc.; however, it is generally about 1 to 40 hours.

The thus obtained 1,2-benzisothiazol-3-one compound represented by formula (2): 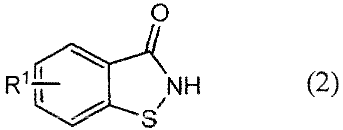 wherein R¹ is the same atom or group as R¹ in formula (1), can be easily isolated and purified, for example, by directly crystallizing from a reaction mixture containing the compound, or extracting and recrystallizing, etc.

Specific examples of the 1,2-benzisothiazol-3-one compounds represented by formula (2) include 1,2-benzisothiazol-3-one, 7-methyl-1,2-benzisothiazol-3-one, 5-butyl-1,2-benzisothiazol-3-one, 6-methoxy-1,2-benzisothiazol-3-one, 7-nitro-1,2-benzisothiazol-3-one, 6-chloro-1,2-benzisothiazol-3-one, 6-carboxy-1,2-benzisothiazol-3-one, and 6-methoxycarboxy-1,2-benzisothiazol-3-one. Among these, 1,2-benzisothiazol-3-one is particularly preferable as it can provides products with high antimicrobial activity.

### (2) Method for reusing by-product

In the present invention, an alkyl halide, which is generated as a by-product in the method for producing a 1,2-benzisothiazol-3-one compound by the method described above, and which is represented by formula (3) : R²X¹, wherein R² is the same as R² in formula (1) and X¹ is a halogen atom, is converted into a 2-(alkylthio)benzonitrile compound represented by formula (1) in step (i) and step (ii) described below, and the resulting product is reused as a starting material for producing a 1,2-benzisothiazol-3-one compound. This method is explained in detail below.

### (i) Synthesis step of alkali metal salt of alkylthiol:

As the first step, an alkyl halide represented by formula (3): R²X¹, wherein R² is the same as R² in formula (1) and X¹ is a halogen atom, which is obtained as a by-product in the production of a 1,2-benzisothiazol-3-one compound, is reacted with at least one sulfide selected from the group consisting of alkali metal hydrogensulfide and alkali metal sulfide to form an alkylthiol represented by formula (4): R²SH, wherein R² is the same as R² in formula (1), and the resulting alkylthiol is reacted with a base containing an alkali metal to convert it into an alkali metal salt of alkylthiol.

In this step, after removing a hydrogen halide, which is present in addition to an alkyl halide as a by-product in the product obtained by the reaction between a 2-(alkylthio)benzonitrile compound and a halogenating agent, the result may be reacted with sulfide. The removal of the hydrogen halide can be performed by, for example, reacting the reaction product containing a hydrogen halide with an aqueous solution containing an alkali metal hydroxide, such as a sodium hydroxide. More specifically, when the reaction product is obtained in the form of a gas, the gas generated by the reaction may be passed through an aqueous solution containing an alkali metal hydroxide.

In formula (3): R²X¹, R² is the same as R² in formula (1), i.e., a C₁₋₄ alkyl group. The halogen atom represented by X¹ corresponds to the halogen atom in the halogenating agent used in the reaction with a compound represented by formula (1). Specific examples thereof include a chlorine atom and a bromine atom.

The alkyl halide represented by formula (3) depends on the combination of the compound represented by formula (1) and the halogenating agent used in the production of 1,2-benzisothiazol-3-one compound. Specific examples thereof include methyl chloride, ethyl chloride, n-propyl chloride, isopropyl chloride, n-butyl chloride, isobutyl chloride, sec-butyl chloride, tert-butyl chloride, methyl bromide, ethyl bromide, n-propyl bromide, isopropyl bromide, n-butyl bromide, isobutyl bromide, sec-butyl bromide, and tert-butyl bromide.

Among the sulfides usable in the reaction with the alkyl halide represented by formula (3), examples of alkali metal sulfide include sodium sulfide and potassium sulfide. Examples of alkali metal hydrogensulfide include lithium hydrogensulfide, sodium hydrogensulfide, potassium hydrogensulfide, rubidium hydrogensulfide, and cesium hydrogensulfide. These alkali metal sulfide and alkali metal hydrogensulfide may be used singly or in a combination of two or more. Among these, alkali metal hydrogensulfide, such as sodium hydrogensulfide and potassium hydrogensulfide, are preferable in view of water solubility and cost effectiveness.

The amount of sulfide is preferably about 0.5 to 3 mol, and more preferably about 0.9 to 2 mol, per mol of the alkyl halide represented by formula (3). When the amount of sulfide is unduly small, the yield may be lowered. When the amount of sulfide is unduly large, an effect commensurate with the amount of sulfide cannot be obtained, and thus it is not economically advantageous. Therefore, both cases are undesirable.

The reaction between the alkyl halide represented by formula (3) and sulfide may be conducted in a homogeneous system using, as a reaction solvent, a polar solvent such as N-methyl pyrrolidone, or dimethyl sulfoxide. However, in order to facilitate the reaction or to facilitate the liquid separation of the product after the reaction, it is preferable that the reaction be performed in the presence of water and a phase transfer catalyst in a heterogeneous system.

In this case, the amount of water may be generally about 1 to 50 mol, and preferably about 5 to 20 mol, per mol of alkyl halide. When the amount of water is unduly small, a salt as by-product is deposited, which may make stirring impossible. The use of an unduly large amount of water is not economically advantageous since a commensurate effect cannot be obtained.

For example, in the case of using a C₁ or C₂ alkyl halide as the starting material, when the starting material has a low boiling point and is in a gaseous form at ordinary temperatures, the reaction is performed by dissolving the starting material in a water-insoluble organic solvent. In the case of using a C₃ or C₄ alkyl halide as the starting material, when the starting material itself is a water-insoluble liquid, the reaction system inherently becomes a two phase system even if water is used alone. However, in order to reduce the volatility of alkyl halide, facilitate the reaction, and facilitate the liquid separation of the product after the reaction, a water insoluble organic solvent is preferably used.

The water-insoluble organic solvent is not particularly limited and usable examples thereof include hydrocarbons, such as n-hexane, n-heptane, cyclohexane, methylcyclohexane, benzene, toluene, and xylene; and halogenated hydrocarbons, such as chloroform, chlorobenzene, and dichlorobenzene. The amount of the water-insoluble organic solvent is preferably about 10 to 300 parts by mass relative to 100 parts by mass of water.

Examples of phase transfer catalysts include quaternary ammonium salts, such as benzyltriethylammonium bromide, benzyltrimethylammonium chloride, hexadecyltriethylammonium bromide, hexadecyltriethylammonium bromide, hexadecyltrimethylammonium chloride, dodecyltriethylammonium chloride, octyltriethylammonium bromide, tetra-n-butylammonium bromide, tetra-n-butylammonium chloride, tetraethylammonium chloride, and trioctylmethylammonium chloride; quaternary phosphonium salts, such as hexadecyltriethylphosphonium bromide, hexadecyltributylphosphonium chloride, tetra-n-butylphosphonium bromide, tetra-n-butylphosphonium chloride, trioctylethylphosphonium bromide, and tetraphenylphosphonium bromide; and crown ethers, such as 18-crown-6, dibenzo-18-crown-6, and dicyclohexyl-18-crown-6. Among these, from the viewpoint of cost effectiveness, quarternary-ammonium-salts, such as tetra-n-butylammonium bromide and tetra-n-butylammonium chloride; quaternary phosphonium salts, such as tetra-n-butylphosphonium chloride; etc., are preferable.

The proportion of the phase transfer catalyst used may be generally in the range of about 0.001 to 0.1 mol, and preferably about 0.005 to 0.02 mol, per mol of alkyl halide. When the amount of phase transfer catalyst is unduly small, a satisfactory catalytic effect cannot be achieved. The use of an unduly large amount of phase transfer catalyst does not achieve an effect commensurate with the amount of phase transfer catalyst used, and thus it is not economically advantageous.

The reaction temperature may be in the range, generally, of about 0 to 120°C, and preferably about 20 to 100°C. When the reaction temperature is too high, side reactions easily occur. When the reaction temperature is too low, the reaction rate becomes too slow for practical use. Therefore, both cases are not preferable.

The reaction time cannot be generalized as it depends on the reaction temperature, kind of phase transfer catalyst, etc. The reaction time is generally within the range of about 1 to 50 hours.

The method described above makes it possible to obtain a reaction solution containing alkylthiol represented by formula (4): R²SH, wherein R² is the same as R² in formula (1). The reaction solution thus obtained can be easily purified and isolated by general treatment, such as liquid separation, extraction, and distillation. Furthermore, because the water phase is separated while containing a phase transfer catalyst, it can be continuously used in a subsequent reaction as is; therefore, the water phase can be repeatedly used.

The types of alkylthiol represented by formula (4) depend on the type of compound represented by formula (1) that is used in the production of a 1,2-benzisothiazol-3-one compound. Specific examples thereof include methanethiol, ethanethiol, n-propanethiol, isopropanethiol, n-butanethiol, isobutanethiol, sec-butanethiol, and tert-butanethiol.

Subsequently, the alkylthiol represented by formula (4) obtained by the reaction described above is reacted with a base containing an alkali metal to obtain an alkali metal salt of alkylthiol.

The reaction method between the alkylthiol represented by formula (4) and a base is not particularly limited. As an example of a preferable method, when the reaction between the alkyl halide represented by formula (3) and a sulfide is conducted in a heterogeneous system, after separating an organic layer containing alkylthiol from the water layer, an aqueous base solution containing the alkali metal is added to the organic layer, and the water layer is separated from the oil layer again to obtain an alkali metal salt of alkylthiol in the form of an aqueous solution.

The concentration of the aqueous base solution may be generally about 0.1 to 45% by weight, and preferably about 5 to 30% by weight. When the concentration is too high, an alkali metal salt of alkylthiol is deposited, complicating the operation. In contrast, when the concentration is too low, the amount of solvent undesirably increases and is thus not economical.

Examples of the bases containing an alkali metal include alkali metal hydroxides, such as sodium hydroxide and potassium hydroxide; alkali metal carbonates, such as sodium carbonate and potassium carbonate; and metal alcoholates, such as sodium methylate and sodium ethylate. Among these, sodium hydroxide is preferable from an economic point of view.

The proportion of the base may be generally in the range of about 0.8 to 3.5 mol, and preferably about 1.0 to 2.5 mol, per mol of alkylthiol represented by formula (4). When the amount of the base used is unduly small, the yield lowers. Even if an unduly large amount of base is used, extra effects commensurate with that amount cannot be obtained, and thus it is not economically advantageous.

### (ii) Process of synthesizing 2-(alkylthio)benzonitrile compound

In this process, the alkali metal salt of alkylthiol represented by formula (4): R²SH, wherein R² is the same as R² in formula (1), which was obtained in step (i) above is reacted with a 2-halobenzonitrile compound represented by formula (5): 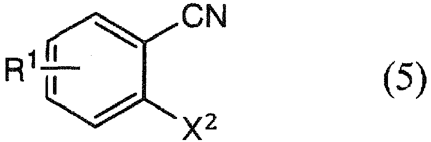 wherein R¹ is a hydrogen atom, a C₁₋₄ alkyl group, a C₁₋₄ alkoxy group, a nitro group, a carboxyl group, an alkoxycarbonyl group, or a halogen atom, and X² is a halogen atom, to obtain a 2-(alkylthio)benzonitrile compound represented by formula (1): 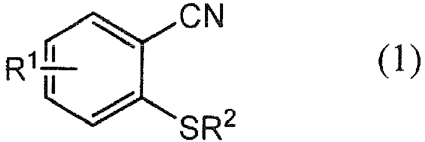 wherein R¹ and R² are as defined above.

The groups represented by R¹ in a 2-halobenzonitrile compound represented by formula (5): 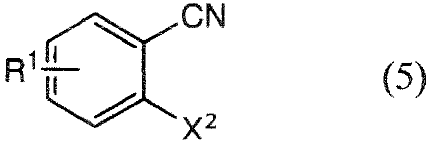 are the same as those represented by R¹ in formula (1). Examples of the halogen atoms represented by X² include a chlorine atom and a bromine atom.

Specific examples of the 2-halobenzonitrile compounds represented by formula (5) include 2-chlorobenzonitrile, 2-bromobenzonitrile, 3-methyl-2-chlorobenzonitrile, 5-butyl-2-chlorobenzonitrile, 4-methoxy-2-chlorobenzonitrile, 2-chloro-3-nitrobenzonitrile, and 4-methoxycarbonyl-2-chlorobenzonitrile. Among these, 2-chlorobenzonitrile is preferable as it is readily available and can provide products with high antimicrobial activity.

The reaction between the alkali metal salt of alkylthiol represented by formula (4) and the 2-halobenzonitrile compound represented by formula (5) is preferably conducted in the presence of water and a phase transfer catalyst in a heterogeneous system.

In the reaction between the alkali metal salt of alkylthiol represented by formula (4) and the 2-halobenzonitrile compound represented by formula (5), the proportions thereof are preferably about 0.5 to 3 mol, and more preferably about 0.9 to 2 mol, of an alkali metal salt of alkylthiol, per mol of a 2-halobenzonitrile compound. When the proportion of the alkali metal salt of alkylthiol is unduly small, the yield may be lowered. When the proportion of the alkali metal salt of alkylthiol is unduly large, any extra effects commensurate with that amount cannot be obtained, and thus it is not economically advantageous.

The proportion of water may be generally in the range of about 1 to 50 mol, and preferably about 5 to 20 mol, per mol of alkali metal salt of alkylthiol. When the proportion of water is unduly small, a salt as by-product is deposited, which may make stirring impossible. The use of an unduly large proportion of water is not economically desirable since a commensurate effect cannot be obtained.

Examples of usable phase transfer catalysts include the same phase transfer catalysts as those used in the reaction between the alkyl halide and sulfide described in Item (i).

The proportion of phase transfer catalyst may be in the range of generally about 0.001 to 0.1 mol, and preferably about 0.005 to 0.02 mol, per mol of an alkali metal salt of alkylthiol. When the proportion of the phase transfer catalyst is unduly small, a sufficient catalytic effect cannot be obtained. The use of an unduly large proportion of phase transfer catalyst is not economically desirable since a commensurate effect cannot be obtained.

The reaction system inherently becomes a two-phase system even if water is used alone. However, in order to facilitate the reaction, or to facilitate the liquid separation of the product after the reaction, a water-insoluble organic solvent is preferably used.

The water-insoluble organic solvent is not particularly limited as long as it is inactive to the reaction. Examples of usable water-insoluble organic solvents include hydrocarbons, such as n-hexane, n-heptane, cyclohexane, methylcyclohexane, benzene, toluene, and xylene; and halogenated hydrocarbons, such as methylene chloride, 1,2-dichloroethane, and chlorobenzene. In the case of using the same solvent as that used in the reaction between a 2-(alkylthio)benzonitrile compound represented by formula (1) and a halogenating agent as the water-insoluble organic solvent in this step, when the 1,2-benzisothiazol-3-one compound represented by formula (2) is produced using the 2-(alkylthio)benzonitrile compound, which has been obtained in this step, as a starting material, the production of the 2-(alkylthio)benzonitrile compound and the production of the 1,2-benzisothiazol-3-one compound can be continuously performed as a one-pot process and the 1,2-benzisothiazol-3-one compound can be efficiently obtained.

The amount of water-insoluble organic solvent is preferably about 10 to 300 parts by mass, per 100 parts by mass of water.

The reaction temperature may be generally in the range of about 0 to 120°C, and preferably in the range of about 20 to 100°C. When the reaction temperature is unduly high, side reactions easily occur. When the reaction temperature is unduly low, the reaction speed becomes too slow for practical use.

The reaction time cannot be generalized as it depends on the reaction temperature, type of phase transfer catalyst, type of reactant, etc. The reaction time is generally in the range of about 1 to 50 hours.

This reaction makes it possible to obtain a 2-(alkylthio)benzonitrile compound represented by formula (1): 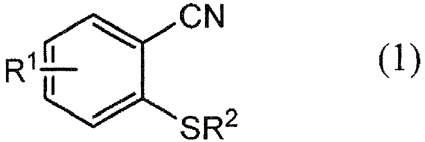 wherein R¹ and R² are as defined above. The solution containing a 2-(alkylthio)benzonitrile compound thus obtained can be isolated and purified by an ordinary process, such as liquid separation, extraction, and distillation. Because the water phase is separated while containing a phase transfer catalyst, it can be continuously used in a subsequent reaction as is; therefore, the water phase can be repeatedly used.

### (iii) Method for reuse

By performing step (i) and step (ii) in a combined manner, the alkyl halide represented by formula (3), which is a by-product generated in the process of producing the 1,2-benzisothiazol-3-one compound represented by formula (2) by reacting the 2-(alkylthio)benzonitrile compound represented by formula (1) with a halogenating agent, can be converted into a 2-(alkylthio)benzonitrile compound represented by formula (1) in a simple manner at a high yield.

By reacting the 2-(alkylthio)benzonitrile compound obtained by this method with a halogenating agent under the conditions described above, the 1,2-benzisothiazol-3-one compound represented by formula (2) can be obtained. This makes it possible to reuse the by-product generated during the formation of the 1,2-benzisothiazol-3-one compound. This also makes it unnecessary to equip a facility for disposal of the by-product or reduces the burden of treating waste liquid, so that the by-product can be effectively reused and a 1,2-benzisothiazol-3-one compound can be obtained in an economical manner.

### Advantageous Effects of Invention

The present invention makes it possible to reuse alkyl halide, which is a by-product generated in the process of producing a 1,2-benzisothiazol-3-one compound, as a starting material for producing a 1,2-benzisothiazol-3-one compound. As a result, a 1,2-benzisothiazol-3-one compound can be produced in an economically advantageous manner with less burden on the environment.

### Description of Embodiments

The present invention is explained in detail below with reference to Examples. However, the scope of the present invention is not limited to these Examples.

### Example 1

### (i) Synthesis of methanethiol

A 20% aqueous solution (280 g) of sodium hydrogensulfide (1.00 mol), chlorobenzene (200 g), and a 50% by weight aqueous solution (3.2 g) of tetra-n-butyl ammonium bromide were placed in a 500-mL four-necked flask equipped with a stirrer, a thermometer, a condenser, and a gas-blowing pipe under a nitrogen atmosphere. The mixture was stirred at 30°C.

2-(Methylthio)benzonitrile (298.0 g, 2.0 mol)? monochlorobenzene (460.0 g), and 35% by weight hydrochloric acid (67.0 g, hydrochloric acid: 0.6 mol, water: 2.4 mol) were placed in a 3-L four-necked flask equipped with a stirrer, a thermometer, and a condenser. Sulfuryl chloride (270.0 g, 2.0 mol) was added thereto while stirring at 5 to 15°C. The mixture was further heated to 65 to 70°C and allowed to react for 1 hour. The gas generated during the reaction was passed through a 15% aqueous solution of sodium hydroxide to remove hydrogen chloride. The resulting chloromethane gas (50.5 g, 1.00 mol) was blown into the solution containing sodium hydrogensulfide obtained above. After blowing, the mixture was further stirred at 30°C for 7 hours to be allowed to react.

After completion of the reaction, the water phase was separated off. A 20% aqueous solution (200 g) of sodium hydroxide was added to the resulting chlorobenzene layer, and the resulting mixture was subjected to extraction by stirring. The chlorobenzene layer was separated off, obtaining an aqueous solution (261 g) of sodium salt of methanethiol. The concentration of the sodium salt of methanethiol was determined by gas chromatography, and the weight of the obtained sodium salt of methanethiol was calculated to find that the weight thereof was 64 g (0.92 mol). The yield of the target product relative to chloromethane was 92%.

### (ii) Synthesis of 2-(methylthio)benzonitrile

2-Chlorobenzonitrile (27.5 g, 0.2 mol), monochlorobenzene (26.0 g), and a 50% by weight aqueous solution (0.5 g) of tetra-n-butyl ammonium bromide were placed in a 0.5-L four-necked flask equipped with a stirrer, a thermometer, a dropping funnel, and a condenser under a nitrogen atmosphere. A 30% by weight aqueous solution (51.4 g) of sodium salt of methanethiol(0.22 mol) was added dropwise at 70 to 75°C over a period of 5 hours. After the completion of dropwise addition, the mixture was further allowed to react at the same temperature for 10 hours.

After completion of the reaction, the reaction mixture was cooled to room temperature. The solvent was distilled off, and then the reaction mixture was distilled under a reduced pressure to obtain a fraction of 139 to 140°C/931Pa, giving 29.2 g (0.196 mol) of 2-(methylthio)benzonitrile. The yield of the target product relative to 2-chlorobenzonitrile was 98%.

### (iii) Synthesis of 1,2-benzisothiazole 3-one

The 2-(methylthio)benzonitrile (29.8 g, 0.2 mol) obtained in Item (ii) above, monochlorobenzene (46.0 g), and 35% by weight hydrochloric acid (6.7 g, hydrochloric acid: 0.06 mol, water: 0.24 mol) were placed in a 0.5-L four-necked flask equipped with a stirrer, a thermometer, and a condenser. While stirring, chlorine (15.6 g, 0.22 mol) was blown therein over a period of 2 hours at 45 to 50°C. The resulting mixture was further heated to 65 to 70°C and then allowed to react for 1 hour. After completion of the reaction, a 20% by weight aqueous solution (41.0 g) of sodium hydroxide was added thereto at the same temperature and the mixture was cooled to room temperature.

The precipitated crystal was collected by filtration, washed with monochlorobenzene, and dried to obtain 1,2-benzisothiazol-3-one (29.6 g, 0.196 mol). The yield of the target product relative to 2-(methylthio)benzonitrile was 98%. The purity of the obtained 1,2-benzisothiazol-3-one measured with high-performance liquid chromatography was 99.5%.

### Example 2

### (i) Synthesis of ethanethiol

A 20% aqueous solution (280 g) of sodium hydrogensulfide (1.00 mol), chlorobenzene (100 g), and a 50% by weight aqueous solution (3.2 g) of tetra-n-butyl ammonium bromide were placed in a 500-mL four-necked flask equipped with a stirrer, a thermometer, a condenser, and a gas-blowing pipe under a nitrogen atmosphere. The mixture was stirred at 30°C.

2-(Ethylthio)benzonitrile (326.0 g, 2.0 mol), monochlorobenzene (460.0 g), and a 35% by weight hydrochloric acid (67.0 g, hydrochloric acid: 0.6 mol, water: 2.4 mol) were placed in a 3-L four-necked flask equipped with a stirrer, a thermometer, and a condenser. While stirring, chlorine (156.0 g, 2.2 mol) was blown therein over a period of 2 hours at 45 to 50°C, the resulting mixture was further heated to 65 to 70°C and then allowed to react for 1 hour. The gas generated during the reaction was passed through a 15% aqueous solution of sodium hydroxide to remove hydrogen chloride. The resulting chloroethane gas (64.5 g, 1.00 mol) was blown into the solution containing sodium hydrogensulfide obtained above. After blowing, the mixture was further stirred at 30°C for 7 hours to be allowed to react.

After completion of the reaction, the water phase was separated off. A 20% aqueous solution (200 g) of sodium hydroxide was added to the resulting chlorobenzene layer. The resulting mixture was subjected to extraction by stirring and the chlorobenzene layer was separated, obtaining an aqueous solution (257 g) of sodium salt of ethanethiol. The concentration of the sodium salt of ethanethiol was determined by gas chromatography, and the weight of the obtained sodium salt of ethanethiol was calculated. As a result, the weight thereof was 74 g (0.88 mol). The yield of the target product relative to chloromethane was 85%.

### (ii) Synthesis of 1,2-benzisothiazol-3-one (one-pot reaction)

2-Chlorobenzonitrile (27.5 g, 0.2 mol), monochlorobenzene (100 g), and a 50% by weight aqueous solution (2.25 g) of tetra-n-butyl ammonium bromide were placed in a 500-mL four-necked flask equipped with a stirrer, a thermometer, and a condenser under a nitrogen atmosphere. A 30% by weight aqueous solution (67.3 g) of the sodium salt of ethanethiol (0.24 mol) obtained in Item (i) above was added to the mixture in the four-necked flask while stirring. The resulting mixture was allowed to react for 2 hours under reflux.

After completion of the reaction, the mixture was separated into a water layer and an oil layer at 40 to 50°C. Monochlorobenzene (100 g) and water (3.6 g, 0.2 mol) were added to the oil layer. After adding sulfuryl chloride (27.0 g, 0.2 mol) at 5 to 15°C while stirring, the mixture was heated to 70 to 80°C and allowed to react for 1 hour.

After completion of the reaction, the reaction mixture was cooled to room temperature. The deposited white crystal was washed with monochlorobenzene and then dried to give 1,2-benzisothiazol-3-one (29.3 g, melting point: 157 to 158°C). The yield of the target product relative to 2-chlorobenzonitrile (starting material) was 97%.

## Claims

1. A method for producing a 1,2-benzisothiazol-3-one compound represented by formula (2): 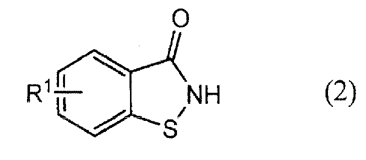 wherein R¹ is a hydrogen atom, a C₁₋₄ alkyl group, a C₁₋₄ alkoxy group, a nitro group, a carboxyl group, an alkoxycarbonyl group, or a halogen atom;
the method comprising reacting a 2-(alkylthio)benzonitrile compound represented by formula (1): 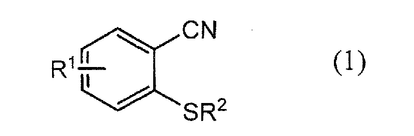 wherein R¹ is as defined above and R² is a C₁₋₄ alkyl group, with a halogenating agent in the presence of water;
wherein an alkyl halide generated as a by-product in the above reaction and represented by formula (3): R²X¹, wherein R² is as defined above and X¹ is a halogen atom, is subjected to step (i) and step (ii) described below to obtain a 2-(alkylthio)benzonitrile compound represented by formula (1) above, and the resulting 2-(alkylthio)benzonitrile compound is reused as a starting material for producing a 1,2-benzisothiazol-3-one compound:
(i) reacting the alkyl halide represented by formula (3): R²X¹, wherein R² and X¹ are as defined above, with at least one sulfide selected from the group consisting of alkali metal hydrogensulfide and alkali metal sulfide to form an alkylthiol represented by formula (4): R²SH, wherein R² is as defined above, and then converting the alkylthiol into an alkali metal salt of alkylthiol; and
(ii) reacting the alkali metal salt of alkylthiol obtained in step (i) with a 2-halobenzonitrile compound represented by formula (5): 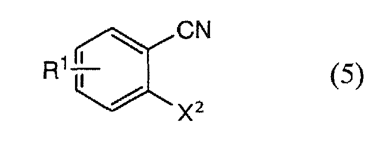 wherein R¹ is as defined above and X² is a halogen atom, to obtain the 2-(alkylthio)benzonitrile compound represented by formula (1).

2. The method according to claim 1, wherein the reaction of step (i) is conducted in the presence of water and a phase transfer catalyst.

3. The method according to claim 1 or 2, wherein the reaction of step (ii) is conducted in the presence of water and a phase transfer catalyst.

4. The method according to any one of claims 1 to 3, wherein the sulfide used in step (i) is an alkali metal hydrogensulfide.

5. The method according to any one of claims 1 to 4, wherein R¹ is a hydrogen atom and R² is a methyl group in formula (1).

6. A method for producing a 2-(alkylthio)benzonitrile compound represented by formula (1): 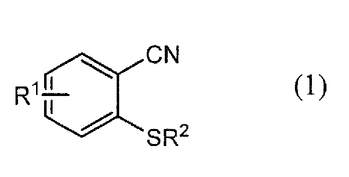 wherein R¹ is a hydrogen atom, a C₁₋₄ alkyl group, a C₁₋₄ alkoxy group, a nitro group, a carboxyl group, an alkoxycarbonyl group, or a halogen atom, and R² is a C₁₋₄ alkyl group;
the method comprising:
reacting an alkyl halide represented by formula (3): R²X¹, wherein R² is as defined above and X¹ is a halogen atom, with at least one sulfide selected from the group consisting of alkali metal hydrogensulfide and alkali metal sulfide to obtain an alkylthiol represented by formula (4): R²SH, wherein R² is as defined above,
converting the alkylthiol into an alkali metal salt of alkylthiol, and then
reacting the resulting alkali metal salt of alkylthiol with a 2-halobenzonitrile compound represented by formula (5): 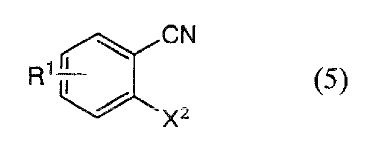 wherein R¹ is as defined above and X² is a halogen atom.
